# EUROPÄISCHE PATENTANMELDUNG

(11) **EP 4 624 014 A1**
(43) Veröffentlichungstag der Anmeldung: **01.10.2025**
(21) Anmeldenummer: 24167223.7
(22) Anmeldetag: 28.03.2024
(51) Int. Cl.: B01D 3/06, B01D 3/10, B01D 5/00, C08F 6/10, C08F 6/12

(54) **VORRICHTUNG UND VERFAHREN ZUR DESTILLATION VON POLYISOCYANATEN**

(71) Anmelder: Covestro Deutschland AG, 51373 Leverkusen (DE)
(72) Erfinder: Drumm, Christian, 66903 Frohnhofen (DE); Schuetze, Mike, 51379 Leverkusen (DE); Meuresch, Markus, 50259 Pulheim (DE); Malbertz, Christoph, 52134 Herzogenrath (DE); Merkel, Michael, 40223 Düsseldorf (DE)
(74) Vertreter: Levpat

(57) **Zusammenfassung**

Die Erfindung betrifft eine Destillationsvorrichtung zur Abtrennung von monomerem Diisocyanat aus einer Polyisocyanatzusammensetzung, umfassend wenigstens eine Vorverdampfungseinrichtung umfassend einen ersten Einlass für die zu destillierende Polyisocyanatzusammensetzung, einen Vorverdampfer mit einem ersten Innenraum, einen ersten Gasauslass für verdampfte Bestandteile, einen ersten Flüssigkeitsauslass für nicht verdampfte Bestandteile der Polyisocyanatzusammensetzung und wenigstens einen dem ersten Gasauslass zugeordneten ersten Kondensator, wenigstens eine Dünnschichtverdampfungseinrichtung umfassend einen zweiten Einlass für die nicht verdampften Bestandteile der Polyisocyanatzusammensetzung, Dünnschichtverdampfer mit einem zweiten Innenraum, einen zweiten Gasauslass für verdampfte Bestandteile, einen zweiten Flüssigkeitsauslass für die von nicht umgesetztem Isocyanat weitgehend befreite Polyisocyanatzusammensetzung und wenigstens einen dem zweiten Gasauslass zugeordneten zweiten Kondensator, sowie eine wenigstens eine Vakuumpumpe umfassende Vakuumerzeugungseinrichtung, die ausgestaltet ist, einen Druck P1 im Innenraum des Vorverdampfers der wenigstens einen Vorverdampfungseinrichtung und einen Druck P2 mit P2<P1 im Innenraum des Dünnschichtverdampfers der wenigstens einen Dünnschichtverdampfungseinrichtung zu erreichen, dadurch gekennzeichnet, dass der erste Flüssigkeitsauslass mit dem zweiten Einlass über eine Verbindungsleitung verbunden ist, die einen Siphon umfasst, der dazu eingerichtet ist, einen Druckabschluss zwischen dem ersten Innenraum und dem zweiten Innenraum herzustellen.

## Beschreibung

Die vorliegende Erfindung betrifft ein Verfahren zur Herstellung monomerenarmer Polyisocyanate sowie eine Vorrichtung zur Destillation von Polyisocyanaten.

Modifizierungsreaktionen aliphatischer und cycloaliphatischer Diisocyanate sind seit langem bekannt. Die dabei erhaltenen Polyisocyanate finden als Vernetzerkomponente in Beschichtungssystemen und Klebstoffen Verwendung. Üblich sind einerseits Modifizierungsreaktionen, in denen die Isocyanate mit sich selbst reagieren und die beispielsweise zur Bildung von Biureten, Isocyanuraten, Uretdionen oder Iminooxadiazindionen führen. Andererseits können die Isocyanate unter Bildung von Urethan-, Allophanat und/oder Harnstoffgruppen mit Poylolen oder Polyaminen umgesetzt und so oligomerisiert werden. Entscheidend ist die Bildung von höhermolekularen Addukten, die einen niedrigeren Dampfdruck aufweisen als die monomeren Diisocyanate selbst. Nicht umgesetztes Diisocyanat wird aus dem Reaktionsgemisch beispielsweise durch Dünnschichtdestillation abgetrennt und es verbleibt das Polyisocyanat als Sumpfprodukt, welches, falls gewünscht, mit Lösungsmittel verdünnt werden kann.

Die katalytische Herstellung von Polyisocyanaten und das destillative Entfernen von nicht umgesetztem Diisocyanat aus einer Polyisocyanat-Mischung ist beispielsweise in WO2008/068198A1 eingehend beschrieben. Das Entfernen des monomeren Diisocyanats erfolgt bevorzugt in einer mehrstufigen Destillation bei Temperaturen zwischen 90 und 220 °C im Vakuum. Der Druck sinkt vorzugsweise von Stufe zu Stufe und erreicht in der letzten Stufe 0,1 bis 10 hPa. Als Apparate kommen Flash-, Fallfilm-, Dünnschicht-, und/oder Kurzwegverdampfer zum Einsatz, wobei letztere wiederum bevorzugt in der letzten Stufe der Destillation zum Einsatz kommen, da sie konstruktionsbedingt einen geringen Druckverlust aufweisen und deshalb einen besonders niedrigen Druck ermöglichen. Auf diese Weise lässt sich das monomere Diisocyanat schonend entfernen. Es wird ausdrücklich darauf hingewiesen, die thermische Belastung möglichst gering zu halten, indem z.B. auf Zwischenbehälter und Vorlagebehälter verzichtet wird oder Sumpfvolumina klein und Rohrleitungen kurz gehalten werden. Das Destillat, also das monomere Diisocyanat wird bevorzugt in die Reaktion zurückgeführt und kann bei Bedarf einer zusätzlichen Behandlung zur Verbesserung der Farbzahl unterworfen werden, beispielsweise einer Filtration. Auf die Kondensation des Destillats und damit verbundene Probleme wird nicht eingegangen.

Auch DE102004038784A1 beschreibt das destillative Entfernen von nicht umgesetztem Diisocyanat aus einer Polyisocyanat-Mischung. Die Destillation erfolgt unter Einsatz mindestens eines Kurzwegverdampfers, gegebenenfalls in Kombination mit anderen Verdampfern. Als Prozessbedingungen wird ein Bereich von 5 mbar bis 10⁻⁴ mbar genannt und eine Verdampfertemperatur von 30 bis 230 °C. In einem Ausführungsbeispiel wird monomeres Diphenylmethandiisocyanat bei 0,05 mbar und einer Verdampfertemperatur von 177 °C destillativ abgetrennt und das verdampfte monomere Diphenylmethandiisocyanat bei 50 °C kondensiert.

EP1426393A2 beschreibt die Herstellung monomerenarmer uretdiongruppenhaltiger Polyisocyanate. In den Ausführungsbeispielen wird das destillative Entfernen von Hexamethylendiisocyanat aus der Polyisocyanatmischung beschrieben. Es erfolgt mittels eines Kurzwegverdampfers mit Vorverdampfer bei einem Druck von 0,1 bis 0,5 mbar und einer Heizmedium-Temperatur von 140 bis 150 °C. Nicht umgesetztes Monomer und der Katalysator werden auf diese Weise abgetrennt und das Destillat erneut zur Reaktion eingesetzt.

Auch die WO2021008908A1 beschreibt ein Verfahren zur Destillation von Polyisocyanaten. Dabei erfolgt die Kondensation der Brüden in wenigstens zwei Stufen bei unterschiedlicher Kondensatortemperatur. Auch die Destillation selbst erfolgt mehrstufig, wobei vorzugsweise der Druck von Stufe zu Stufe gesenkt wird. Der Druck in der ersten Stufe liegt vorzugsweise im Bereich von 2 bis 100 mbar und der Druck in der letzten Stufe vorzugsweise zwischen 0,005 und 10 mbar. Das Ausführungsbeispiel beschreibt ein dreistufiges Vakuumsystem mit je einer Vakuumpumpe für jede Destillationsstufe, wobei die Vakuumpumpen in Reihe installiert sind. Die Apparate sind dabei wie folgend verschaltet: Die erste Destillationsstufe mit einem Druck von 25 mbar ist zwischen der zweiten und der dritten Vakuumpumpe eingebunden, die zweite Destillationstufe mit einem Druck von 7 mbar zwischen der ersten und der zweiten Vakuumpumpe und die letzte Destillationsstufe mit einem Druck von 1,4 mbar auf der Saugseite der ersten Vakuumpumpe.

Zusammenfassend lässt sich feststellen, dass die destillative Abtrennung von monomerem Diisocyanat aus Polyisocyanaten einen sehr niedrigen Druck benötigt. Aufgrund der teilweise recht hohen Monomergehalte der Rohwaren erfolgt die Destillation im industriellen Maßstab in der Regel mehrstufig, wobei in den einzelnen Stufen unterschiedliche Drücke herrschen und vor allem die letzte Stufe, die nur noch mit wenig Monomer beaufschlagt wird, bei einem sehr tiefen Druck betrieben wird. Eine einstufige Destillation im industriellen Maßstab wäre durch den Dampfdruck des Monomers limitiert und würde darüber hinaus sehr große Apparate fordern, die wiederum nur schwer auf das nötige Druckniveau gebracht werden können und außerdem mit sehr hohen Investitionskosten einhergehen.

Kommt es zu Druckschwankungen im Destillationssystem, ergeben sich hieraus im Betrieb Nachteile. Auf der einen Seite kommt es zu einem verstärkten Mitriss von Tröpfchen des höher siedenden Polyisocyanats mit dem abdestillierten Monomer. Dies führt nicht nur zu Ausbeuteverlusten, sondern auch zu hartnäckigen Ablagerungen am Kopf der jeweiligen Destillationsapparate sowie in den sich anschließenden Rohrleitungen und Apparaten, wie beispielsweise Kondensatoren. Auch kann es, sofern das Destillat wieder zur Herstellung von weiterem Polyisocyanat eingesetzt wird, zu Qualitätseinbußen kommen. Auf der anderen Seite wird die Abtrennung des Monomers aus dem Polyisocyanat beeinträchtigt, so dass der Restmonomergehalt im Polyisocyanat unter sonst gleichen Bedingungen zu hoch ausfällt.

Es hat sich nun gezeigt, dass derartige Druckschwankungen sich bei der Destillation von Polyisocyanaten in einem mehrstufigen Destillationssystem aus einem oder mehreren Vorverdampfern und einem Dünnschichtverdampfer häufig auf eine unzureichende Trennung der Druckstufen zurückführen lassen. Grundsätzlich existieren zahlreiche Möglichkeiten, diese Trennung umzusetzen, wobei viele die beschriebenen Probleme entweder nicht vermeiden oder mit anderen Nachteilen einhergehen.

Es bestand also die Aufgabe ein Verfahren und eine Vorrichtung zur mehrstufigen Destillation von Polyisocyanaten bereitzustellen, wobei eine zuverlässige und wartungsarme Trennung der Druckstufen vorliegt, die einen konstanten Betrieb ermöglicht. Hierzu wurden im Rahmen der vorliegenden Erfindung verschiedene Überlegungen und Versuche durchgeführt.

Als einfache und übliche Maßnahme ist die Installation eines federbelasteten Druckhalteventils in der Verbindungsleitung des letzten Vorverdampfers und des Dünnschichtverdampfers denkbar. Der in der Regel recht geringe Druckunterschied, der unterhalb der gängigen Arbeitsbereiche solcher Ventile liegt, führt bei dieser Maßnahme jedoch zu Problemen. Selbst dann, wenn sich ein Druckhalteventil mit passendem Arbeitsbereich findet, führt dieses im Betrieb zu Fluktuationen im Zulaufstrom des Dünnschichtverdampfers, der auf derartige Schwankungen sensibel reagiert. Außerdem besteht die Gefahr, dass bewegliche Teile des Druckhalteventils auf Dauer durch höhermolekulare Anteile der Polyisocyanatzusammensetzung verklebt werden, was die Funktion beeinträchtigt.

Eine weitere Variante ist die Implementierung einer Standregelung im Abscheider des Vorverdampfers mit einem Regelventil. Auf diese Weise lässt sich der Abfluss der nicht verdampften Anteile der Polyisocyanatzusamensetzung derart Regeln, dass ein gewisser Flüssigkeitsstand im Sumpf des Abscheiders verbleibt und für den gewünschten Druckabschluss sorgt, während Fluktuationen im Vergleich zur Verwendung eines Druckhalteventils reduziert sind. Diese Maßnahme ist einerseits teuer, da entsprechende Sensoren und Aktoren für die Umsetzung benötigt werden, sie ist aber vor allem auch Fehleranfällig. Im Dauerbetrieb kommt es immer wieder zu Problemen mit der Standmessung und zum Verkleben oder Verstopfen des Regelventils durch höhermolekulare Anteile der Polyisocyanatzusammensetzung.

Entsprechendes gilt auch für die Möglichkeit, eine Standregelung beispielsweise mit einer Zahnradpumpe umzusetzen, die häufig für eine gleichmäßige und pulsationsarme Förderung eingesetzt wird und einen Druckabschluss darstellen kann. Auch hier können bewegliche Teile durch das Fördermedium verklebt werden. Da es sich um eine zwangsfördernde Pumpe handelt, tritt darüber hinaus auch das Risiko eines unkontrollierten Druckaufbaus auf, das durch entsprechende Maßnahmen abzusichern ist, also die Investitionskosten und den Wartungsaufwand noch weiter erhöht. Zusätzlich ist zum Beispiel durch eine Füllstandsregelung vor der Pumpe sicherzustellen, dass die Pumpe nicht trocken betrieben wird.

Die Verwendung einer Lochblende, die den Abfluss der nicht verdampften Anteile der Polyisocyanatzusammensetzung drosselt ist ebenfalls denkbar. Der Vorteil dieser Lösung ist, dass sie ohne bewegliche Teile oder aufwändige Regelungstechnik auskommt. Nachteilig ist aber, dass auch hier der mitunter geringe Druckunterschied eine Auslegung der Lochblende erschwert und dass deren Funktion vom Durchsatz abhängig ist und nur in einem sehr engen Lastbereich funktioniert, was für die betrieblichen Praxis unerwünscht ist.

Die Aufgabe konnte überraschenderweise gelöst werden, indem in die Verbindungsleitung zwischen dem letzten Vorverdampfer und dem Dünnschichtverdampfer ein Siphon integriert wird. Dieser ermöglicht einen freien und konstanten Abfluss der nicht verdampften Anteile der Polyisocyanatzusammensetzung während diese gleichzeitig selbst als Sperrflüssigkeit fungieren kann und den Druckabschluss herstellt. Der Siphon funktioniert weitgehend Lastunabhängig und kommt dabei ohne bewegliche Teile aus. Die Auslegung für den angestrebten Druckunterschied erfolgt einfach über die Höhe, die im Siphon für die Sperrflüssigkeitssäule zur Verfügung gestellt wird.

Es können verschiedene Arten von Siphons eingesetzt werden. Geeignet sind beispielsweise Röhrensiphons, die meist U-förmig ausgeführt sind, Flaschensiphons, Tauchwandsiphons oder Glockensiphons. Röhrensiphons haben gegenüber den anderen genannten Bauformen den Vorteil, dass sie weniger zur Ablagerung von Sinkstoffen neigen, gehen aber mit einem höheren Platzbedarf einher. Unabhängig von der Form des verwendeten Siphons ist es vorteilhaft, wenn dieser am tiefsten Punkt oder zumindest in der Nähe des tiefsten Punkts ein Entleerungsventil aufweist.

Somit ist ein erster Gegenstand der Erfindung eine Destillationsvorrichtung zur Abtrennung von monomerem Diisocyanat aus einer Polyisocyanatzusammensetzung, umfassend oder bestehend aus
a. wenigstens eine Vorverdampfungseinrichtung umfassend einen ersten Einlass für die zu destillierende Polyisocyanatzusammensetzung, einen Vorverdampfer mit einem ersten Innenraum, einen ersten Gasauslass für verdampfte Bestandteile, einen ersten Flüssigkeitsauslass für nicht verdampfte Bestandteile der Polyisocyanatzusammensetzung und wenigstens einen dem ersten Gasauslass zugeordneten ersten Kondensator,
b. wenigstens eine Dünnschichtverdampfungseinrichtung umfassend einen zweiten Einlass für die nicht verdampften Bestandteile der Polyisocyanatzusammensetzung, einen Dünnschichtverdampfer mit einem zweiten Innenraum, einen zweiten Gasauslass für verdampfte Bestandteile, einen zweiten Flüssigkeitsauslass für die von monomerem Diisocyanat weitgehend befreite Polyisocyanatzusammensetzung und wenigstens einen dem zweiten Gasauslass zugeordneten zweiten Kondensator, sowie
c. eine wenigstens eine Vakuumpumpe umfassende Vakuumerzeugungseinrichtung, die ausgestaltet ist, einen Druck P1 im Innenraum des Vorverdampfers der wenigstens einen Vorverdampfungseinrichtung und einen Druck P2 mit P2<P1 im Innenraum des Dünnschichtverdampfers der wenigstens einen Dünnschichtverdampfungseinrichtung zu erreichen,
dadurch gekennzeichnet, dass der erste Flüssigkeitsauslass mit dem zweiten Einlass über eine Verbindungsleitung verbunden ist, die einen Siphon umfasst, der dazu eingerichtet ist, einen Druckabschluss zwischen dem ersten Innenraum und dem zweiten Innenraum herzustellen.

Das Wort "*ein*" ist im Rahmen dieser Erfindung im Zusammenhang mit zählbaren Größen lediglich als unbestimmter Artikel und nur dann als Zahlwort zu verstehen, wenn dies ausdrücklich, etwa durch den Zusatz "*genau* ein" gesagt wird. Ausdrücke wie "eine Leitung" oder "eine Vakuumpumpe" schließen also die Möglichkeit des Vorhandenseins weiterer Leitungen oder Vakuumpumpen nicht aus.

Bevorzugt bedeuten die Ausdrücke "umfassend" oder "enthaltend" erfindungsgemäß "im Wesentlichen bestehend aus" und besonders bevorzugt "bestehend aus".

Vorliegend steht "Polyisocyanat" für ein Isocyanat, welches durch Modifizierung aus einem Diisocyanat hergestellt wurde, wobei wenigstens zwei Diisocyanatmoleküle in das Polyisocyanat eingebaut wurden. Solche Polyisocyanate werden im Folgenden auch als Polyisocyanatzusammensetzung und allgemein häufig auch als Lack-Polyisocyanate bezeichnet. "Polyisocyanat" steht also ausdrücklich nicht für ein Isocyanat, wie es direkt aus der Phosgenierungsreaktion eines Di- oder Polyamins erhalten wird. Solche Isocyanate, die aus der Phosgenierung erhalten werden, werden vorliegend als Monomere Isocyanate oder monomere Diisocyanate bezeichnet.

Die Vorverdampfungseinrichtung, die Dünnschichtverdampfungseinrichtung, die Vakuumerzeugungseinrichtung sowie die Verbindungsleitung können unabhängig voneinander weitere Apparate umfassen, die für den Betrieb der Destillationsvorrichtung dienlich sind, wie beispielsweise Vorlagebehälter, Wärmetauscher, Mischeinrichtungen, Fördereinrichtungen, Inertisierungseinrichtungen, Ablassbehälter oder Regelungstechnik.

In einer bevorzugten Ausführungsform der erfindungsgemäßen Destillationsvorrichtung ist der Siphon als Röhrensiphon, Flaschensiphon, Tauchwandsiphon oder Glockensiphon ausgeführt, besonders bevorzugt ist der Siphon als Röhrensiphon oder Glockensiphon ausgeführt, ganz besonders bevorzugt als Röhrensiphon. Der Siphon ist dabei vorzugsweise dazu eingerichtet, eine Sperrflüssigkeitshöhe h von wenigstens 25 mm, bevorzugt wenigstens 50 mm, besonders bevorzugt wenigstens 100 mm und ganz besonders bevorzugt wenigstens 150 mm zu erreichen. Grundsätzlich ist es auch möglich, in die Verbindungsleitung mehrere Siphons (Siphon 1, Siphon 2, ..., bis Siphon n) in Reihe zu integrieren, was beispielsweise bei begrenzten Platzverhältnissen Vorteile bieten kann. In diesem Fall ist als relevante Sperrflüssigkeitshöhe h die Summe der jeweiligen Sperrflüssigkeitshöhen h(Siphon 1) + h(Siphon 2) + ... + h(Siphon n) anzusetzen. Vorzugsweise ist der Siphon und/oder andere Teile der Verbindungsleitung beheizbar ausgeführt, verfügt also beispielsweise über einen Doppelmantel oder eine elektrische Begleitheizung.

Auch ist es möglich, die Verbindungsleitung nicht als einzelne Verbindungsleitung zu konstruieren, sondern zwei oder mehr Leitungen zu verwenden, die nebeneinander verlaufen. In diesem Fall sind im Sinne der Erfindung alle Leitungen mit jeweils wenigstens einem Siphon für den Druckabschluss auszustatten.

Bevorzugt ist die Ausführungsform mit genau einer Verbindungsleitung, die über genau einen Siphon für den Druckabschluss verfügt.

Bei dem Vorverdampfer handelt es sich beispielsweise um einen Fallfilmverdampfer, Umlaufverdampfer, Flash-Verdampfer, Plattenverdampfer oder Kesselverdampfer. Aufgrund der schonenden Betriebsbedingungen sind Fallfilmverdampfer, bei denen die Verdampfung aus einem dünnen Film und damit schon bei geringen Temperaturunterschieden zwischen der Verdampferoberfläche und der Polyisocyanatzusammensetzung erfolgt, besonders bevorzugt. Besonders geeignete Fallfilmverdampfer sind beispielsweise Rohrbündel-Fallrohrverdampfer mit mehreren, im Wesentlichen senkrecht angeordneten, im Wesentlichen parallel verlaufenden Verdampferrohren. Es ist auch möglich, mehrere Vorverdampfer in der Vorverdampfungseinrichtung zu kombinieren. Dies kann wahlweise parallel erfolgen oder in wenigstens zwei in Reihe geschalteten Stufen, wobei die am Sumpf eines Vorverdampfers entnommene Flüssigkeit dann als Zulauf für einen weiteren Vorverdampfer dient.

Der Vorverdampfer verfügt üblicherweise über einen Verteiler, der für eine gleichmäßige Verteilung der zu destillierenden Polyisocyanat-Zusammensetzung auf die Heizflächen des Vorverdampfers sorgt. Optional kann der erste Gasauslass des Vorverdampfers mit einem Tröpfchenabscheider ausgerüstet sein, um den Mitriss von Polyisocyanat-Tröpfchen mit dem Destillat zu verringern.

Dem ersten Gasauslass ist wenigstens ein Kondensator zugeordnet, der dazu eingerichtet ist, die Brüden des Vorverdampfers enthaltend monomeres Diisocyanat weitgehend zu kondensieren. In einer bevorzugten Ausführungsform sind dem ersten Gasauslas wenigstens zwei, besonders bevorzugt genau zwei Kondensatoren zugeordnet, die derart mit dem Vorverdampfer und der Vakuumerzeugungseinrichtung verbunden sind, dass die Brüden aus dem Vorverdampfer auf ihrem Weg zum Vakuumsystem die Kondensatoren nacheinander durchlaufen. Zweckmäßig weist dabei jeder folgende Kondensator die gleiche oder eine niedrigere, bevorzugt eine niedrigere Temperatur auf als der vorhergehende. Geeignete Kondensatoren sind insbesondere Rohrbündelwärmetauscher. In der Regel erfolgt die Kondensation außerhalb des Vorverdampfers, so dass vorliegend von externen oder außenliegenden Kondensatoren gesprochen wird. Es ist aber auch denkbar, einen oder mehrere Kondensatoren als interne (oder "innenliegende") Kondensatoren in den Vorverdampfer zu integrieren, der dann einen separaten Flüssigkeitsabzug für die kondensierten Brüden aufweist.

Geeignete Dünnschichtverdampfer sind beispielsweise Starrflügelrotor-Dünnschichtverdampfer, Wischerklappendünnschichtverdampfer oder Dünnschicht-Kurzwegverdampfer.

Der Dünnschichtverdampfer verfügt üblicherweise über einen Verteiler, der für eine gleichmäßige Verteilung der Polyisocyanat-Zusammensetzung auf die Heizflächen des Dünnschichtverdampfers sorgt. Optional kann der zweite Gasauslass, also der Gasauslass des Dünnschichtverdampfers mit einem Tröpfchenabscheider ausgerüstet sein, um den Mitriss von Polyisocyanat-Tröpfchen mit dem Destillat zu verringern.

Dem zweiten Gasauslass ist wenigstens ein Kondensator zugeordnet, der dazu eingerichtet ist, die Brüden des Dünnschichtverdampfers enthaltend monomeres Diisocyanat weitgehend zu kondensieren. In einer bevorzugten Ausführungsform sind dem zweiten Gasauslas wenigstens zwei, besonders bevorzugt genau zwei oder drei Kondensatoren zugeordnet, die derart mit dem Dünnschichtverdampfer und der Vakuumerzeugungseinrichtung verbunden sind, dass die Brüden aus dem Dünnschichtverdampfer auf ihrem Weg zum Vakuumsystem die Kondensatoren nacheinander durchlaufen. Zweckmäßig weist dabei jeder folgende Kondensator die gleiche oder eine niedrigere, bevorzugt eine niedrigere Temperatur auf als der vorhergehende. Geeignete Kondensatoren sind insbesondere Rohrbündelwärmetauscher. In der Regel erfolgt die Kondensation außerhalb des Vorverdampfers, so dass vorliegend von externen oder außenliegenden Kondensatoren gesprochen wird. Es ist aber auch denkbar, einen oder mehrere Kondensatoren als interne (oder "innenliegende") Kondensatoren in den Vorverdampfer zu integrieren, der dann einen separaten Flüssigkeitsabzug für die kondensierten Brüden aufweist. Über einen solchen internen (oder "innenliegenden") Kondensator verfügen beispielsweise Kurzwegverdampfer, die deshalb über einen besonders niedrigen Druckverlust verfügen und für Anwendungen mit besonders hohen Anforderungen an das Vakuum bevorzugt eingesetzt werden.

Die Vakuumerzeugungseinrichtung ist über Rohrleitungen oder Schläuche, bevorzugt Rohrleitungen, mit der Vorverdampfungseinrichtung und der Dünnschichtverdampfungseinrichtung verbunden. Sie umfasst wenigstens eine Vakuumpumpe. Die Vakuumerzeugungseinrichtung ist dazu eingerichtet, Gas aus dem ersten Innenraum des Vorverdampfers durch den ersten Gasauslass und Gas aus dem zweiten Innenraum des Dünnschichtverdampfers durch den zweiten Gasauslass abzuziehen. Dabei ist das Vakuumsystem erfindungsgemäß ausgestaltet, im ersten Innenraum des Vorverdampfers einen Druck P1 und im zweiten Innenraum des Dünnschichtverdampfers einen Druck P2 mit P2 < P1 zu erreichen. Bevorzugt ist die erfindungsgemäße Vorrichtung ausgestaltet, einen Druck P1 im Innenraum des Vorverdampfers im Bereich von 100 Pa bis 12000 Pa, bevorzugt 200 Pa bis 4000 Pa, besonders bevorzugt 300 Pa bis 2500 Pa zu erreichen, wobei gilt, dass der zu erreichende Druck P1 oberhalb des Drucks P2 im zweiten Innenraum des Dünnschichtverdampfers ist. Ebenfalls bevorzugt ist die erfindungsgemäße Vorrichtung ausgestaltet, einen Druck P2 im zweiten Innenraum des Dünnschichtverdampfers im Bereich von 0,1 Pa bis 2000 Pa, bevorzugt 5 Pa bis 1000 Pa, besonders bevorzugt 10 Pa bis 100 Pa zu erreichen, wobei gilt, dass der zu erreichende Druck P2 unterhalb des Drucks P1 im Innenraum des Vorverdampfers ist. Ganz besonders bevorzugt ist die erfindungsgemäße Vorrichtung ausgestaltet, P1 und P2 in den vorgenannten Bereichen zu erreichen, wobei gilt, dass P2 < P1 ist und die zu erreichende Differenz dP = P1 - P2 im Bereich von 50 Pa bis 10000 Pa, bevorzugt 80 Pa bis 2500 Pa ist.

Als Vakuumpumpen kommen grundsätzlich die dem Fachmann für diese Anwendung bekannten Pumpen oder Verdichter in Frage wie beispielsweise Membranpumpen, Hubkolbenpumpen, Drehschieberpumpen, Sperrschieberpumpen, Wälzkolbenpumpen, Schraubenverdichter oder Flüssigkeitsringverdichter. Bevorzugt werden als Vakuumpumpen Wälzkolbenpumpen und/oder Flüssigkeitsringverdichter verwendet. Besonders bevorzugt ist eine Kombination aus einer Vorvakuumpumpe und einer oder mehreren Hauptvakuumpumpen, wobei als Vorvakuumpumpe bevorzugt ein Flüssigkeitsringverdichter und als Hauptvakuumpumpe oder -pumpen eine oder mehrere Wälzkolbenpumpen verwendet werden.

In einer weiteren bevorzugten Ausführungsform der erfindungsgemäßen Vorrichtung ist dem zweiten Flüssigkeitsauslass der Dünnschichtverdampfungseinrichtung, also dem Auslass für die durch Destillation von monomerem Diisocyanat weitgehend befreite Polyisocyanatzusammensetzung ein Wärmetauscher nachgeschaltet, der dazu eingerichtet ist, diese Zusammensetzung abzukühlen, bevorzugt rasch abzukühlen. Vorzugsweise ist der Wärmetauscher derart dimensioniert, dass die von monomerem Diisocyanat weitgehend befreite Polyisocyanatzusammensetzung nach dem Verlassen des Dünnschichtverdampfers in weniger als 5 Minuten, besonders bevorzugt weniger als 1 Minute auf eine Temperatur unterhalb von 100 °C, bevorzugt unterhalb von 98 °C und besonders bevorzugt unterhalb von 96 °C abkühlt. Durch eine Temperaturregelung kann sichergestellt werden, dass die von monomerem Diisocyanat weitgehend befreite Polyisocyanatzusammensetzung nicht zu stark abgekühlt wird. Bevorzugt sind Wärmetauscher und Temperaturregelung dazu eingerichtet, die von monomerem Diisocyanat weitgehend befreite Polyisocyanatzusammensetzung auf eine Temperatur im Bereich von 50 °C bis 100 °C, besonders bevorzugt 60 °C bis 98 °C und ganz besonders bevorzugt 70 °C bis 96 °C abzukühlen. Als Wärmetauscher für die Abkühlung eignen sich beispielsweise Plattenwärmetauscher, Rohrbündelwärmetauscher oder Doppelrohrwärmetauscher.

Ein weiterer Gegenstand der Erfindung ist ein Verfahren zur Abtrennung von monomerem Diisocyanat aus einer Polyisocyanatzusammensetzung durch wenigstens zweistufige Destillation in einer Destillationsvorrichtung umfassend einen Vorverdampfer und einen Dünnschichtverdampfer, dadurch gekennzeichnet, dass
- im Innenraum des Vorverdampfers ein Druck P1 vorliegt und
- im Innenraum des Dünnschichtverdampfers ein Druck P2 vorliegt, für den gilt P2<P1,
wobei eine an monomerem Diisocyanat abgereicherte Polyisocyanatzusammensetzung als Sumpfprodukt des Vorverdampfers durch eine Verbindungsleitung umfassend einen Siphon in den Dünnschichtverdampfer geleitet wird.

In einer bevorzugten Ausführungsform des erfindungsgemäßen Verfahrens fungiert die ablaufende, an monomerem Diisocyanat abgereicherte Polyisocyanatzusammensetzung selbst im Siphon als Sperrflüssigkeit.

In einer weiteren bevorzugten Ausführungsform des erfindungsgemäßen Verfahrens liegt der Druck P1 im Bereich von 100 Pa bis 12000 Pa, bevorzugt von 200 Pa bis 4000 Pa und besonders bevorzugt 300 Pa bis 2500 Pa, und/oder die Temperatur des Vorverdampfers im Bereich von 110 °C bis 200 °C, bevorzugt von 120 °C bis 190 °C und besonders bevorzugt von 135 °C bis 175 °C sowie der Druck P2 im Bereich von 0,1 Pa bis 2000 Pa, bevorzugt von 5 Pa bis 1000 Pa und besonders bevorzugt 10 Pa bis 100 Pa und/oder die Temperatur des Dünnschichtverdampfers im Bereich von 120 °C bis 220 °C, bevorzugt von 135 °C bis 190 °C und besonders bevorzugt von 140°C bis 175 °C, wobei die Differenz dP = P1 - P2 im Bereich von 50 Pa bis 10000 Pa, bevorzugt 80 Pa bis 2500 Pa liegt.

Der Siphon in der Verbindungsleitung dient der Trennung der Vakuumstufen im Vorverdampfer und im Dünnschichtverdampfer. Da er ohne bewegliche Teile auskommt, ist er besonders für den vorliegenden Einsatzweck in der Destillation von Polyisocyanaten geeignet. Um weitgehend unabhängig von Lastzuständen, Druck- und Temperaturschwankungen oder auch sich ändernden Zusammensetzungen des Feedstroms zu funktionieren, ist es bevorzugt, dass die Sperrflüssigkeit im Siphon einen Druckabschluss mit einer Sperrflüssigkeitshöhe h von wenigstens 25 mm, bevorzugt wenigstens 50 mm, besonders bevorzugt wenigstens 100 mm und ganz besonders bevorzugt wenigstens 150 mm ausbildet. Je höher der Druckunterschied zwischen den beiden Destillationsstufen ist, desto höher sollte auch die Sperrflüssigkeitshöhe des/der Siphons ausgelegt werden. Für die Sperrflüssigkeitshöhe h gilt vorzugsweise, dass der Wert der Sperrflüssigkeitshöhe h in Millimetern mindestens dem 10-fachen Wert des Druckunterschieds dP in Pascal entspricht. Ist diese Bedingung erfüllt, verlieren andere Parameter, z.B. der Druckverlust in der Verbindungsleitung, an Bedeutung für die Auslegung der Destillationsvorrichtung. Eine unzureichende Trennung der Vakuumstufen kann Druckschwankungen und daraus resultierende Probleme zur Folge haben. So kann es einerseits zum unkontrollierten Sieden im Vorverdampfer kommen, wobei verstärkter Mitriss von Flüssigkeit in den Kondensator auftritt und andererseits kann monomeres Diisocyanat aus dem Vorverdampfer in den Dünnschichtverdampfer durchschlagen und diesen überlasten, so dass die gewünschte Produktqualität nicht zuverlässig erreicht wird.

Bei dem Polyisocyanat bzw. der Polyisocyanatzusammensetzung handelt es sich beispielsweise um das Rohprodukt einer Isocyanat-Oligomerisierung, welches noch Anteile an nicht umgesetztem monomerem Diisocyanat enthält. Die Oligomerisierung kann dabei sowohl durch Reaktion eines monomeren Diisocyanats mit sich selbst oder aber mit weiteren Verbindungen wie beispielsweise Polyolen, Polythiolen oder Polyaminen erfolgen. Bevorzugt erfolgt die Umsetzung in Anwesenheit wenigstens eines Katalysators und wird beim Erreichen eines bestimmten Umsetzungsgrades durch Desaktivierung des Katalysators gestoppt. Die Desaktivierung kann auf verschiedene Arten erfolgen. Bevorzugt erfolgt sie durch Zugabe einer Verbindung, die den jeweiligen Katalysator desaktiviert. Manche Katalysatoren lassen sich auch thermisch Desaktivieren oder aus dem Reaktionsgemisch entfernen, um die Reaktion zu stoppen. Je nach durchgeführter Reaktion enthält also die Polyisocyanatzusammensetzung vorzugsweise Uretdiongruppen, Isocyanuratgruppen, Urethangruppen, Harnstoffgruppen, Biuretgruppen, Iminooxadiazindiongruppen, Allophanatgruppen oder Kombinationen dieser funktionellen Gruppen, besonders bevorzugt Isocyanuratgruppen, Urethangruppen, Biuretgruppen, Iminooxadiazindiongruppen, oder Kombinationen dieser funktionellen Gruppen und ganz besonders bevorzugt Isocyanuratgruppen und/oder Urethangruppen. Die Kombination der Gruppen kann dabei innerhalb einzelner Oligomer-Moleküle vorliegen und/oder als Mischung von Molekülen mit unterschiedlichen funktionellen Gruppen.

Als abzutrennende monomere Diisocyanate kommen generell alle industriell verfügbaren aliphatischen, cycloaliphatischen, araliphatischen oder aromatischen Diisocyanate in Betracht, die über einen geringeren Siedepunkt bzw. bei gegebener Temperatur über einen höheren Dampfdruck verfügen als das Polyisocyanat, von dem sie abgetrennt werden sollen. Vorzugsweise handelt es sich bei dem abzutrennenden monomeren Diisocyanat um das Disocyanat, aus dem durch Oligomerisierung das Polyisocyanat entstanden ist, so dass die Polyisocyanatzusammensetzung im Wesentlichen das Polyisocyanat und nicht umgesetztes monomeres Diisocyanat sowie gegebenenfalls Reste von Hilfsstoffen wie Katalysatoren, Lösungsmitteln und/oder Stoppern umfasst oder bevorzugt daraus besteht. Besonders bevorzugt handelt es sich um Diisocyanate ausgewählt ist aus der Gruppe bestehend aus 1,5-Diisocyanatopentan (PDI), 1,6-Diisocyanatohexan (HDI), 1-Isocyanato-3,3,5-trimethyl-5-isocyanatomethylcyclohexan (Isophorondiisocyanat, IPDI), 4,4'-Diisocyanatodicyclohexylmethan, 2,4'-Diisocyanatodicyclohexylmethan, Bis-(isocyanatomethyl)-norbornan, 1,3- und 1,4-Bis(isocyanatomethyl)benzol (XDI), 2,4- und 2,6-Toluylendiisocyanat (folgend einzeln oder gemeinsam Toluylendiisocyanat oder TDI genannt), 2,4'- und 4,4'-Diisocyanatodiphenylmethan (MDI) oder um ein Gemisch zweier oder mehrerer Diisocyanate aus dieser Gruppe. Besonders bevorzugt handelt es sich bei dem abzutrennenden monomeren Diisocyanat um ein Diisocyanat ausgewählt aus der Gruppe bestehend aus 1,5-Diisocyanatopentan, 1,6-Diisocyanatohexan, Isophorondiisocyanat und Toluylendiisocyanat oder eine Mischung zweier oder mehrerer Diisocyanate aus dieser Gruppe. Ganz besonders bevorzugt handelt es sich um Toluylendiisocyanat oder eine Mischung aus Toluylendiisocyanat und 1,6-Diisocyanatohexan oder eine Mischung aus Toluylendiisocyanat und 1,5-Diisocyanatopentan.

Das erfindungsgemäße Verfahren eignet sich vor allem zur Abtrennung von monomeren Diisocyanaten aus Polyisocyanatzusammensetzungen, wobei die Polyisocyanatzusammensetzung vor der Destillation bevorzugt einen Gehalt an monomerem Diisocyanat (Monomergehalt) von wenigstens 40 Gew.-%, bevorzugt wenigstens 50 Gew.-% und besonders bevorzugt wenigstens 55 Gew.-% bezogen auf die gesamte Polyisocyanatzusammensetzung aufweist.

Aus dieser Polyisocyanatzusamensetzung wird im Vorverdampfer monomeres Isocyanat durch Destillation teilweise entfernt, so dass am Sumpf des Vorverdampfers eine an monomer abgereicherte Polyisocyanatzusamensetzung in flüssiger Form anfällt. Diese an monomerem Diisocyanat abgereicherte Polyisocyanatzusammensetzung verlässt den Vorverdampfer als Sumpfprodukt und weist bevorzugt einen Gehalt an monomerem Diisocyanat von 1,0 Gew-% bis 18,0 Gew.-%, bevorzugt von 3,0 Gew.-% bis 15,0 Gew.-% und besonders bevorzugt von 5,0 Gew.-% bis 12,0 Gew.-% bezogen auf die ab monomerem Diisocyanat abgereicherte Polyisocyanatzusammensetzung auf. Restgehalte an monomerem Diisocyanat in diesem Bereich sind besonders vorteilhaft, weil damit einerseits sichergestellt ist, dass die Viskosität der an monomerem Diisocyanat abgereicherten Polyisocyanatzusammensetzung nicht zu hoch ist, so dass ein besserer Austausch der Sperrflüssigkeit im Siphon erfolgt und eine Bildung hochmolekularer Ablagerungen verhindert wird und andererseits der Dünnschichtverdampfer nicht durch zu viel Monomer überlastet wird, was sich aufgrund höherer Druckverluste beim Abdestillieren des Restmonomers im Dünnschichtverdampfer negativ auf das Vakuum in dessen Innenraum auswirken würde.

Die anfallende an monomerem Isocyanat abgereicherte Polyisocyanatzusammensetzung wird durch die Verbindungsleitung als Feedstrom in den Dünnschichtverdamfper geleitet. Sie passiert dabei den Siphon, der so konstruiert ist, dass dabei bevorzugt ein Teil der ablaufenden Polyisocyanatzusammensetzung im Siphon verbleibt und einen Druckabschluss mit der Sperrflüssigkeitshöhe h ausbildet. Die Polyisocyanatzusammensetzung fungiert bevorzugt also selbst als Sperrflüssigkeit im Siphon und stellt einen Druckabschluss zwischen dem ersten Innenraum des Vorverdampfers und dem zweiten Innenraum des Dünnschichtverdampfers her. Dabei erfolgt ein ständiger zumindest teilweiser Austausch der Sperrflüssigkeit, so dass diese eine weitgehend konstante Qualität aufweist und nur minimal durch Alterungsprozesse, wie beispielsweise einen weiteren Molekulargewichtsaufbau der Polyisocyanat-Bestandteile, beeinträchtigt wird. Sofern doch Gas durch die Sperrflüssigkeit in Richtung des Dünnschichtverdampfers perlt, passiert dieses kontrolliert, gleichmäßig und in geringem Ausmaß, so dass die hervorgerufenen Druckschwankungen in beiden Innenräumen gering sind und sich nur unwesentlich auf die Qualität des destillierten Produkts auswirken.

Im Dünnschichtverdampfer wird die Abtrennung von monomerem Diisocyanat aus der Polyisocyanatzusammensetzung weitgehend vervollständigt. Vorzugsweise enthält die als Sumpfprodukt anfallende, von monomerem Diisocyanat weitgehend befreite Polyisocyanatzusammensetzung maximal 0,5 Gew.-% an monomeren Diisocyanaten bezogen auf die gesamte Masse der Polyisocyanatzusammensetzung. Besonders bevorzugt beträgt dieser Gehalt maximal 0,3 Gew.-% und ganz besonderes bevorzugt maximal 0,1 Gew.-%.

In einer bevorzugten Ausführungsform der Erfindung wird der Sumpfablauf nach dem Austritt aus der Dünnschichtverdamfpungsvorrichtung abgekühlt, bevorzugt rasch abgekühlt. Bevorzugt wird der Sumpfablauf dabei auf eine Temperatur im Bereich von 50 °C bis 100 °C, besonders bevorzugt 60 °C bis 98 °C und ganz besonders bevorzugt 70 °C bis 96 °C abgekühlt. Auf diese Weise kann eine Polyisocyanatzusammensetzung erhalten werden, die nur noch sehr geringe Gehalte an monomerem Diisocyanat aufweist. Ohne an eine Theorie gebunden zu sein, wird vermutet, dass auf diese Weise die Rückspaltung von Polyisocyanat-Bestandteilen und damit die neuerliche Bildung von monomerem Diisocyanat verhindert oder zumindest minimiert wird. Es ist bevorzugt, die Abkühlung aktiv z.B. in einem Wärmetauscher oder durch das Quenchen mit bereits gekühltem Produkt direkt nach dem Austrag aus dem Dünnschichtverdampfer durchzuführen. Die Verweildauer des Produkts bei Temperaturen oberhalb von 100 °C, bevorzugt oberhalb von 98 °C und besonders bevorzugt oberhalb von 96 °C sollte nach dem Verlassen des Dünnschichtverdampfers bevorzugt weniger als 5 Minuten, besonders bevorzugt weniger als 1 Minute betragen, um ein besonders monomerenarmes Produkt zu erhalten.

Das erfindungsgemäße Verfahren kann auch als Verfahren zur Herstellung einer an monomer abgereicherten Polyisocyanatzusammensetzung ausgestaltet sein, umfassend den weiteren Schritt der Überführung in eine Produktvorlage, eine Abfüllvorrichtung oder in einen Mischbehälter, in dem das Produkt beispielsweise mit Lösungsmittel abgemischt wird.

Mit der erfindungsgemäßen Destillationsvorrichtung und dem erfindungsgemäßen Verfahren lassen sich effizient Polyisocyanatzusammensetzungen bereitstellen, die vielfältig weiterverwendet werden können. Ein weiterer Gegenstand der vorliegenden Erfindung ist die Verwendung einer erfindungsgemäßen Destillationsvorrichtung in der Herstellung von Klebstoffzusammensetzungen oder Ein- oder Zweikomponenten-Polyurethanlacken. Die Vorteile der Erfindung kommen bei der Verwendung für hochviskose Polyisocyanatzusammensetzungen noch stärker zum Tragen, da ohne bewegliche Teile im erfindungswesentlichen Siphon eine deutlich reduzierte Gefahr von Verklebungen solcher beweglicher Teile und damit verbesserte Sicherheit und Anlageneffizienz erreicht werden kann.

Bei der Verwendung als Vernetzerkomponente in Klebstoffen oder Zweikomponenten-Beschichtungen werden die Polyisocyanatzusammensetzungen in der Regel mit OH- und/oder NH-Komponenten kombiniert, wie sie aus Zweikomponenten-Polyurethansystemen an sich bekannt sind, so z. B. hydroxyfunktionellen Polyestern, Polyacrylaten, Polycarbonaten, Polyethern, Polyurethanen sowie polyfunktionellen Aminen. Sie können aber auch einkomponentig zur Herstellung (anteilig) feuchtigkeitshärtender Kunststoffe und Beschichtungen verwendet werden.

Bevorzugt ist die Verwendung der erfindungsgemäßen Destillationsvorrichtung in der Herstellung von Verbundkörpern umfassend wenigstens eine ausgehärtete Polyisocyanatzusammensetzung, die in direktem Kontakt mit wenigstens einem Substrat ausgewählt aus der Gruppe bestehend aus Metall, Kunststoff, Holz oder deren Mischungen, steht.

Die vorliegende Erfindung wird im Folgenden anhand der Figuren sowie der nachfolgenden Beispiele erörtert, ist jedoch nicht auf diese beschränkt. Es zeigen:
**FIG. 1** schematisch eine Ausfiihrungsform der erfindungsgemäßen Destillationsvorrichtung.
**FIG. 2A, FIG. 2B, FIG.2C** und **FIG. 2D** schematisch verschiedene Ausführungsformen des Siphons **5.**

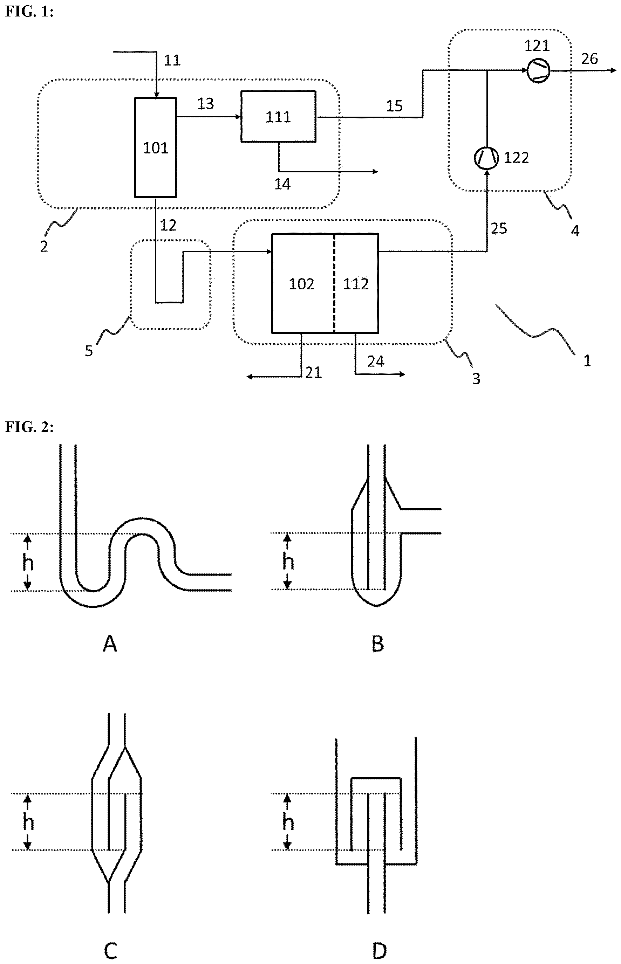

In **FIG. 1** ist schematisch eine Ausführungsform der erfindungsgemäßen Destillationsvorrichtung **1** zur Abtrennung von monomerem Diisocyanat aus einer Polyisocyanatzusammensetzung dargestellt.

Die Destillationsvorrichtung **1** besteht aus einer Vorverdampfungseinrichtung **2** mit einem Vorverdampfer **101**, einer Zulaufleitung **11** für die Polyisocyanatzusammensetzung und einer am Flüssigkeitsauslass abgehenden Verbindungsleitung **12** mit einem Siphon **5.** Stromabwärts des Siphons **5** mündet die Verbindungsleitung **12** in eine Dünnschichtverdampfungsvorrichtung **3** und dort in einen Dünnschichtverdampfer **102.** Der Dünnschichtverdampfer **102** weist einen Flüssigkeitsauslass auf, von dem eine Leitung **21** für die von monomerem Diisocyanat weitgehend befreite Polyisocyanatzusammensetzung als Produkt abgeht. Die Leitung **21** kann beispielsweise in eine Produktvorlage, eine Abfüllvorrichtung oder in einen Mischbehälter führen, in dem das Produkt beispielsweise mit Lösungsmittel abgemischt wird. Gasseitig ist der Vorverdampfer **101** durch eine Brüdenleitung **13** mit dem Kondensator **111** verbunden und dieser wiederum durch eine Leitung **15** mit der Vakuumerzeugungseinrichtung **4.** Leitung **15** ist dabei zwischen den beiden Vakuumpumpen **121** und **122** eingebunden. Vom Kondensator **111** geht Leitung **14** für das kondensierte Destillat ab. Der Dünnschichtverdampfer **102** ist mit einem innenliegenden Kondensator **112** ausgestattet. Dem Kondensator **112** ist über einen Gasauslass eine Ableitung **25** für nicht kondensierte Brüden zugordnet. Die Ableitung **25** führt auf der Saugseite zur Vakuumpumpe **122** der Vakuumerzeugungseinrichtung **4.** Der Vakuumerzeugungseinrichtung **4** ist eine Abgasleitung **26** zugeordnet. Kondensiertes Destillat aus dem Kondensator **112** wird durch Leitung **24** abgeleitet. Das durch die Leitungen **14** und **24** abgeleitete kondensierte Destillat kann beispielsweise als Einsatzstoff für die weitere Herstellung von Polyisocyanatzusammensetzung verwendet werden.

Bei Betrieb der in **FIG. 1** beschriebenen Destillationsvorrichtung **1** wird zur Abtrennung von monomerem Diisocyanat aus einer Polyisocyanatzusammensetzung zunächst die Polyisocyanatzusammensetzung über die Zulaufleitung **11** in den Vorverdampfer **101** der Vorverdampfungseinrichtung **2** geleitet und darin in eine gasförmige und eine flüssige Phase aufgetrennt.

Die flüssige Phase verlässt den Vorverdampfer **101** über die Verbindungsleitung **12** hin zum Dünnschichtverdampfer **102** der Dünnschichtverdampfungsvorrichtung **3.** Im Siphon **5** der Verbindungsleitung **12** bildet die flüssige Phase beim Durchfluss einen Druckabschluss. Die gasförmige Phase aus dem Vordampfer **101** verlässt diesen über die Brüdenleitung **13** hin zum Kondensator **111** und wird dort zumindest teilweise kondensiert. Das kondensierte Destillat wird über Leitung **14** abgeführt und der verbleibende Gasstrom wird über Leitung **15** in die Vakuumerzeugungseinrichtung **4** geleitet. Die über Verbindungsleitung **12** in den Dünnschichtverdampfer **102** geführte flüssige Phase wird darin weiter gereinigt und verlässt den Dünnschichtverdampfer als von monomerem Diisocyanat weitgehend befreite Polyisocyanatzusammensetzung über Leitung **21.** Dieses Produkt kann beispielsweise in einer Produktvorlage, einer Abfüllvorrichtung oder in einem Mischbehälter, in dem das Produkt beispielsweise mit Lösungsmittel abgemischt wird, gesammelt werden. Die in der Dünnschichtverdampfungseinrichtung **3** nicht kondensierten Brüden verlassen den Kondensator **112** über Ableitung **25** hin zur Saugseite der Vakuumpumpe **122.** Das Abgas aus der Vakuumpumpe **121** verlässt die Vakuumerzeugungseinrichtung **4** über die Abgasleitung **26.** Im Kondensator **112** wird ein Destillat erhalten und das kondensierte Destillat über Leitung **24** aus der Dünnschichtverdampfungseinrichtung **3** abgeleitet. Die kondensierten Destillate aus Leitung **14** und **24** können beispielsweise als Einsatzstoff für die weitere Herstellung von Polyisocyanatzusammensetzungen verwendet werden.

In **FIG. 2** sind verschiedene Ausführungsformen des Siphons **5** schematisch dargestellt.

So zeigt **FIG. 2A** einen Röhrensiphon, **FIG. 2B** einen Flaschensiphon, **FIG. 2C** einen Tauchwandsiphon und **FIG. 2D** einen Glockensiphon.

### Beispiele

Alle Prozentangaben beziehen sich, soweit nichts Anderslautendes vermerkt ist, auf das Gewicht.

Die Rest-Monomeren Gehalte wurden nach gaschromatographisch mit internem Standard gemessen und beziehen sich jeweils auf die Summe aus HDI und TDI.

### Beispiel 1:

Eine Mischung aus Toluol-2,4-diisocyanat (TDI) und 1,6-Diisocyanatohexan (HDI) wurde in Gegenwart von Tributylphosphin als Katalysator trimerisiert. Nach dem Abstoppen wurde ein Rohprodukt erhalten, das noch 60 Gew.-% an monomeren Diisocyanaten (Summe aus TDI und HDI) enthielt.

Dieses Rohprodukt wurde dann in einer Destillationsvorrichtung gemäß FIG. 1 destilliert, wobei in der ersten Stufe ein Fallrohrverdampfer mit nachgeschaltetem Gas-Flüssigkeits-Abscheider verwendet wurde. Die Vorverdampfungseinrichtung wurde bei 145 °C betrieben und es herrschte im Innenraum ein Druck von ca. 300 Pa. Das am Sumpfauslass anfallende Polyisocyanat hatte noch einen Restgehalt von ca. 10 Gew.-% an monomeren Diisocyanaten (Summe aus TDI und HDI). Der Ablauf erfolgte durch eine Verbindungsleitung mit einem Flaschensiphon, dessen konstruktiv vorgesehene Sperrflüssigkeitshöhe 25 mm betrug. Die Verbindungleitung mündete in einem Dünnschichtverdampfer mit einem mechanischen Wischersystem. Dort wurde die Destillation bei einer Temperatur von 162 °C vervollständigt, wobei der gemessene Druck im Dünnschichtverdampfer nur geringen Fluktuationen im Bereich zwischen 10 Pa und 100 Pa unterlag. Die Destillation konnte über mehrere Monate betrieben werden, ohne dass Probleme im Sumpfablauf des Vorverdampfers auftraten, die einen Wartungsstillstand erfordert hätten. Im destillierten Sumpfprodukt stellte sich im zeitlichen Mittel ein Restmonomergehalt von ca. 0,45 Gew.-% ein.

### Beispiel 2

Die Destillationsvorrichtung aus Beispiel 1 wurde modifiziert. Dazu wurde die Verbindungsleitung mit Flaschensiphon gegen eine neue Verbindungsleitung mit Röhrensiphon getauscht. Dieser hatte eine konstruktiv vorgesehene Sperrflüssigkeitshöhe von 150 mm.

Die im Beispiel 1 beobachtete geringfügige Fluktuation des Drucks konnte im Dünnschichtverdampfer in dieser angepassten Vorrichtung weiter reduziert werden, so dass der gemessene Druck im Dünnschichtverdampfer sich nunmehr im Bereich zwischen 10 Pa und 20 Pa bewegte. Dies hatte auch einen positiven Effekt auf die Produktqualität, so dass der Restmonomergehalt im destillierten Sumpfprodukt nun bei konstant 0,30 Gew.-% bis 0,40 Gew.-% lag.

### Beispiel 3

Als weitere Veränderung wurde ausgehend von Beispiel 2 die Temperatur des Vorverdampfers auf 153 °C angehoben. Auf diese Weise konnte die Produktqualität weiter verbessert werden und der Restmonomergehalt im destillierten Sumpfprodukt konnte auf 0,15 Gew.-% im zeitlichen Mittel gesenkt werden.

### Beispiel 4

Als weitere Veränderung wurde ausgehend von Beispiel 3 ein Wärmetauscher (Produktkühler) im Produktablauf des Dünnschichtverdampfers installiert, mit dem das ablaufende Harz rasch in weniger als 1 Minute auf eine Temperatur von 94 °C abgekühlt wurde. Auf diese Weise konnte die Produktqualität weiter verbessert werden und es konnten Restmonomergehalte von weniger als <0,1 Gew.-% im destillierten Sumpfprodukt erreicht werden.

## Patentansprüche

1. Destillationsvorrichtung zur Abtrennung von monomerem Diisocyanat aus einer Polyisocyanatzusammensetzung, umfassend
• wenigstens eine Vorverdampfungseinrichtung umfassend einen ersten Einlass für die zu destillierende Polyisocyanatzusammensetzung, einen Vorverdampfer mit einem ersten Innenraum, einen ersten Gasauslass für verdampfte Bestandteile, einen ersten Flüssigkeitsauslass für nicht verdampfte Bestandteile der Polyisocyanatzusammensetzung und wenigstens einen dem ersten Gasauslass zugeordneten ersten Kondensator,
• wenigstens eine Dünnschichtverdampfungseinrichtung umfassend einen zweiten Einlass für die nicht verdampften Bestandteile der Polyisocyanatzusammensetzung, Dünnschichtverdampfer mit einem zweiten Innenraum, einen zweiten Gasauslass für verdampfte Bestandteile, einen zweiten Flüssigkeitsauslass für die von nicht umgesetztem Isocyanat weitgehend befreite Polyisocyanatzusammensetzung und wenigstens einen dem zweiten Gasauslass zugeordneten zweiten Kondensator, sowie
• eine wenigstens eine Vakuumpumpe umfassende Vakuumerzeugungseinrichtung, die ausgestaltet ist, einen Druck P1 im Innenraum des Vorverdampfers der wenigstens einen Vorverdampfungseinrichtung und einen Druck P2 mit P2<P1 im Innenraum des Dünnschichtverdampfers der wenigstens einen Dünnschichtverdampfungseinrichtung zu erreichen,
**dadurch gekennzeichnet, dass** der erste Flüssigkeitsauslass mit dem zweiten Einlass über eine Verbindungsleitung verbunden ist, die einen Siphon umfasst, der dazu eingerichtet ist, einen Druckabschluss zwischen dem ersten Innenraum und dem zweiten Innenraum herzustellen.

2. Destillationsvorrichtung gemäß Anspruch 1, **dadurch gekennzeichnet, dass** der Siphon als Röhrensiphon, Flaschensiphon, Tauchwandsiphon oder Glockensiphon, bevorzugt als Röhrensiphon oder Glockensiphon und besonders bevorzugt als Röhrensiphon ausgeführt ist.

3. Destillationsvorrichtung gemäß einem der Ansprüche 1 oder 2, **dadurch gekennzeichnet, dass** der Siphon eingerichtet ist, eine Sperrflüssigkeitshöhe h von wenigstens 25 mm, bevorzugt wenigstens 50 mm, besonders bevorzugt wenigstens 100 mm und ganz besonders bevorzugt wenigstens 150 mm zu erreichen.

4. Destillationsvorrichtung gemäß einem der Ansprüche 1 bis 3, **dadurch gekennzeichnet, dass** der Vorverdampfer ein Fallfilmverdampfer, Umlaufverdampfer, Flash-Verdampfer, Plattenverdampfer oder Kesselverdampfer, bevorzugt ein Rohrbündel-Fallrohrverdampfer ist.

5. Destillationsvorrichtung gemäß einem der Ansprüche 1 bis 4, **dadurch gekennzeichnet, dass** der Dünnschichtverdampfer ein Starrflügelrotor-Dünnschichtverdampfer, WischerklappenDünnschichtverdampfer oder Dünnschicht-Kurzwegverdampfer ist.

6. Verfahren zur Abtrennung von monomerem Diisocyanat aus einer Polyisocyanatzusammensetzung durch wenigstens zweistufige Destillation in einer Destillationsvorrichtung umfassend einen Vorverdampfer und einen Dünnschichtverdampfer, **dadurch gekennzeichnet, dass**
• im Innenraum des Vorverdampfers ein Druck P1 vorliegt und
• im Innenraum des Dünnschichtverdampfers ein Druck P2 vorliegt, für den gilt P2<P1,
wobei eine an monomerem Diisocyanat abgereicherte Polyisocyanatzusammensetzung als Sumpfprodukt des Vorverdampfers durch eine Verbindungsleitung umfassend einen Siphon in den Dünnschichtverdampfer geleitet wird.

7. Verfahren gemäß Anspruch 6, **dadurch gekennzeichnet, dass** die ablaufende, an monomerem Diisocyanat abgereicherte Polyisocyanatzusammensetzung selbst im Siphon als Sperrflüssigkeit fungiert.

8. Verfahren gemäß einem der Ansprüche 6 oder 7, **dadurch gekennzeichnet, dass**
• der Druck P1 im Bereich von 100 Pa bis 12000 Pa liegt und/oder im Vorverdampfer eine Temperatur im Bereich von 110 °C bis 200 °C vorliegt,
• der Druck P2 im Bereich von 0,1 Pa bis 2000 Pa liegt und/oder eine Temperatur im Bereich von 120 °C bis 220 °C vorliegt und dass
• die Differenz dP = P1 - P2 im Bereich von 50 Pa bis 10000 Pa liegt

9. Verfahren gemäß einem der Ansprüche 6 bis 8, **dadurch gekennzeichnet, dass** die Sperrflüssigkeit im Siphon einen Druckabschluss mit einer Sperrflüssigkeitshöhe h von wenigstens 25 mm, bevorzugt wenigstens 50 mm, besonders bevorzugt wenigstens 100 mm und ganz besonders bevorzugt wenigstens 150 mm ausbildet.

10. Verfahren gemäß einem der Ansprüche 6 bis 9, **dadurch gekennzeichnet, dass** die Polyisocyanatzusammensetzung Uretdiongruppen, Isocyanuratgruppen, Urethangruppen, Harnstoffgruppen, Biuretgruppen, Iminooxadiazindiongruppen, Allophanatgruppen oder Kombinationen dieser funktionellen Gruppen enthält.

11. Verfahren gemäß einem der Ansprüche 6 bis 10, **dadurch gekennzeichnet, dass** das abzutrennende monomere Diisocyanat ausgewählt ist aus der Gruppe bestehend aus 1,5-Diisocyanatopentan (PDI), 1,6-Diisocyanatohexan (HDI), 1-Isocyanato-3,3,5-trimethyl-5-isocyanatomethylcyclohexan (Isophorondiisocyanat, IPDI), 4,4'-Diisocyanatodicyclohexylmethan, 2,4'-Diisocyanatodicyclohexylmethan, Bis-(isocyanatomethyl)-norbornan, 1,3- und 1,4-Bis(isocyanatomethyl)benzol (XDI), Toluylendiisocyanat (TDI), 2,4'- und 4,4'-Diisocyanatodiphenylmethan (MDI) oder dass es sich um ein Gemisch zweier oder mehrerer Diisocyanate aus dieser Gruppe handelt.

12. Verfahren gemäß einem der Ansprüche 6 bis 11, **dadurch gekennzeichnet, dass** die Polyisocyanatzusammensetzung vor der Destillation einen Monomergehalt von wenigstens 40 Gew.-% bezogen auf das die gesamte Polyisocyanatzusammensetzung aufweist.

13. Verfahren gemäß einem der Ansprüche 6 bis 12, **dadurch gekennzeichnet, dass** die den Vorverdampfer als Sumpfprodukt verlassende, an monomer abgereicherte Polyisocyanatzusammensetzung einen Monomergehalt von 1,0 Gew-% bis 18,0 Gew.-% bezogen auf die Polyisocyanatzusammensetzung aufweist.

14. Verfahren gemäß einem der Ansprüche 6 bis 13, **dadurch gekennzeichnet, dass** das am Sumpf des Dünnschichtverdampfers abgenommene Produkt abgekühlt wird, wobei vorzugsweise die Verweildauer des Sumpfprodukts bei Temperaturen oberhalb von 100 °C, bevorzugt oberhalb von 98 °C und besonders bevorzugt oberhalb von 96 °C nach dem Austritt aus dem Dünnschichtverdampfer maximal 5 Minuten, bevorzugt maximal 1 Minute, beträgt.
